(19) Europäisches Patentamt
European Patent Office
Office européen des brevets

(11) Numéro de publication : **0 195 731 B1**

# (12) FASCICULE DE BREVET EUROPÉEN

(45) Date de publication du fascicule du brevet : **24.01.90**

(51) Int. Cl.$^5$ : **A 23 L 1/237**

(21) Numéro de dépôt : **86420072.0**

(22) Date de dépôt : **13.03.86**

## (54) Nouveaux agents édulcorants, procédé pour édulcorer des produits divers et compositions contenant de tels agents édulcorants.

(30) Priorité : **19.03.85 FR 8504241**

(43) Date de publication de la demande : **24.09.86 Bulletin 86/39**

(45) Mention de la délivrance du brevet : **24.01.90 Bulletin 90/04**

(84) Etats contractants désignés : **AT BE CH DE FR GB IT LI LU NL SE**

(56) Documents cités :
EP-A- 0 107 597
FR-A- 1 554 088
FR-A- 2 087 843

(73) Titulaire : **Nofre, Claude**
**119, Cours Albert Thomas**
**F-69003 Lyon (FR)**

**Tinti, Jean-Marie**
**5, avenue de Grenoble**
**F-69330 Meyzieu (FR)**

(72) Inventeur : **Nofre, Claude**
**119, Cours Albert Thomas**
**F-69003 Lyon (FR)**
Inventeur : **Tinti, Jean-Marie**
**5, avenue de Grenoble**
**F-69330 Meyzieu (FR)**

(74) Mandataire : **Laurent, Michel et al**
**Cabinet LAURENT 20, rue Louis Chirpaz B.P. 32**
**F-69131 Ecully Cedex (FR)**

EP 0 195 731 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

L'invention concerne de nouveaux agents édulcorants. Elle se rapporte également à leur utilisation pour édulcorer des produits divers. Elle couvre enfin les compositions contenant de tels agents édulcorants.

Dans la demande de brevet européen EP-A-0 107 597 de la Déposante, on a décrit, en tant que nouveaux composés chimiques et agents édulcorants, des composés de formule générale :

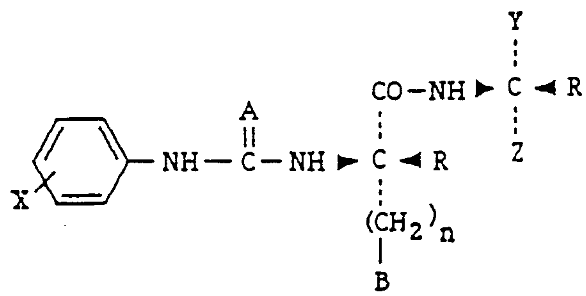

dans laquelle A représente un atome de soufre ou d'oxygène, un groupe imino ou méthylène.

Or, on a observé que ces composés, quand leur radical A est un atome de soufre, ont une stabilité parfois insuffisante, pour certaines utilisations comme agents édulcorants, notamment pour leur utilisation dans des boissons ; il se produit en effet en solution une lente hydrolyse des groupes thiocarbonyle de ces composés. On a également observé que ces composés, quand leur radical A est un groupe imino, sont peu solubles dans l'eau, ce qui est une gêne pour leur utilisation comme agents édulcorants ; de plus, ces mêmes dérivés ont un pouvoir édulcorant limité, ce qui entraîne une augmentation de leur prix de revient.

L'invention pallie ces inconvénients. Elle concerne des agents édulcorants de formule générale :

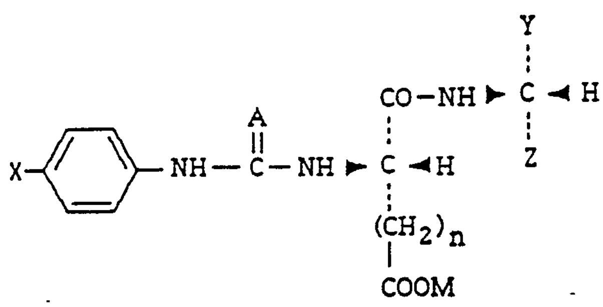

dans laquelle :

X est un substituant CN, $COOC_1$-$C_3$ alkyle, $COC_1$-$C_3$ alkyle, $CONHC_1$-$C_3$ alkyle, $SO_2C_1$-$C_3$ alkyle, $SOC_1$-$C_3$ alkyle, $SO_2NHC_1$-$C_3$ alkyle, $NO_2$, F, Cl ;

n est un nombre égal à 0 ou à 1 ;

M est un atome d'hydrogène, ou un cation organique ou inorganique physiologiquement acceptable ;

Y est un substituant $COOC_1$-$C_3$ alkyle, $C_1$-$C_3$ alkyle, $CH_2OH$, $CHOHCH_3$, $CF_3$ ;

Z est un groupe $C_1$-$C_5$ n-alkyle, isobutyle, isopentyle, phényle, cyclohexyle, benzyle, cyclohexylméthyle, $CH_2C_6H_4OH$(p), $CH_2OC_1$-$C_4$ alkyle, $CH_2COOC_1$-$C_4$ alkyle, $CH_2SC_1$-$C_4$ alkyle, $CH_2CH_2SCH_3$, $CH_2CH_2SO_2CH_3$, $COOC_1$-$C_4$ alkyle, $COOC_3$-$C_7$ cycloalkyle, $CONHC_2$-$C_4$ alkyle, $CONHC_3$-$C_7$ cycloalkyle, $CONHC_3$-$C_7$ thiacycloalkyle, $CONHCH_2COOCH_3$.

Les agents édulcorants selon l'invention se caractérisent en ce que A est un groupe N-CN.

En d'autres termes, la présente invention se distingue du document cité dans le préambule par le choix particulier du radical A, qui est ici un groupe N-CN. Cette substitution est d'autant plus originale et inattendue qu'il n'a jamais été proposé jusqu'ici d'utiliser un tel groupe N-CN pour obtenir des propriétés édulcorantes, et que ce groupe n'est pas chimiquement équivalent aux groupes précédemment utilisés. On sait par ailleurs que toute modification, même légère, de la structure moléculaire d'un agent édulcorant peut entraîner une suppression du caractère édulcorant, et ce d'autant que les relations entre la structure et l'activité édulcorante sont totalement imprévisibles (M.G.J. BEETS, Structure-Activity

Relationships in Human Chemoreception, Applied Science Publ., London, 1978, p. 259-362).

Avantageusement, dans les agents édulcorants selon l'invention :

X est CN, $COOCH_3$, $COOC_2H_5$, $COCH_3$, $CONHCH_3$, $SO_2CH_3$, $SO_2NHCH_3$ ;

n est égal à 1 ;

M est un atome d'hydrogène ou un cation $Na^+$, $K^+$, $NH_4^+$, 1/2 $Ca^{U}$2+, 1/2 $Mg^{2+}$ ;

Y est $COOCH_3$, $CH_3$, $CH_2OH$ ;

Z est un groupe butyle, pentyle, isobutyle, isopentyle, phényle, cyclohexyle, benzyle, cyclohexylméthyle, propylamide, 2-butylamide, dicyclopropylcarbinylamide, 2,2,4,4-tétraméthylthiétan-3-ylamide.

Les agents édulcorants préférés de l'invention sont essentiellement :

les dérivés du N-[cyanoimino(4-X-phénylamino) méthyl]-L-aspartyl-L-phénylalanine méthyl ester, de formule :

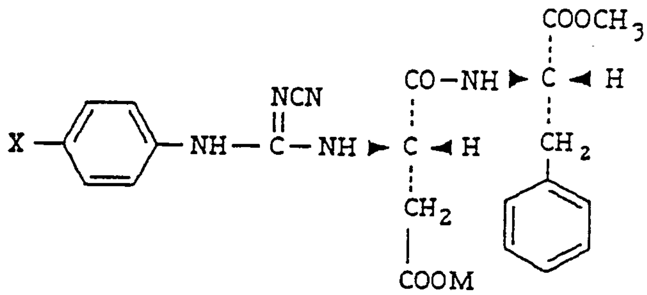

les dérivés de l'acide N-[cyanoimino(4-X-phénylamino)méthyl]-L-aspartique N-[(R)-α-méthylbenzyl] α-monoamide, de formule :

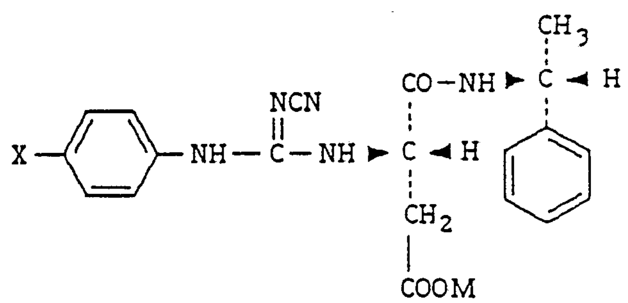

les dérivés du N-[cyanoimino(4-X-phénylamino) méthyl]-L-aspartyl-D-alanine N-(2-butyl)amide, de formule :

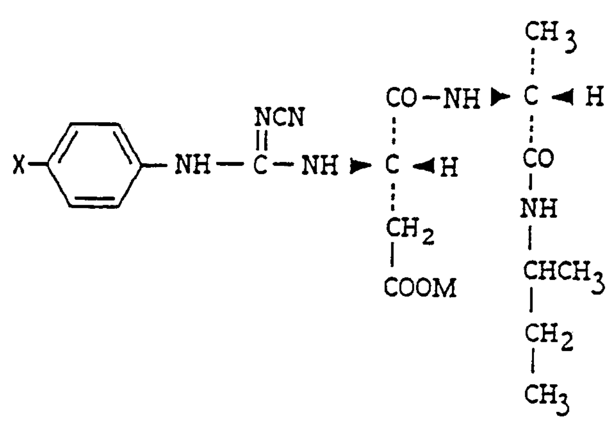

Les composés selon l'invention peuvent être préparés par condensation entre des composés de formules générales suivantes :

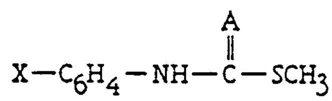

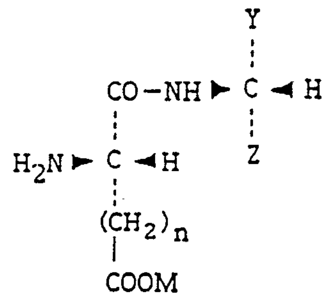

La réaction entre les deux composés peut être effectuée en présence d'une base, la base étant choisie dans le groupe comprenant l'hydroxyde de sodium, l'hydroxyde de potassium, ou une amine tertiaire comme, par exemple, la triéthylamine.

La condensation est effectuée à ébullition dans un mélange éthanol-eau.

L'invention concerne également des compositions édulcorantes contenant de tels composés, seuls ou en mélange avec d'autres composés édulcorants et ce, dans des proportions suffisantes pour être physiologiquement acceptables et efficaces.

La manière dont l'invention peut être réalisée et les avantages qui en découlent ressortiront mieux des exemples de réalisation qui suivent.

### Exemple 1

Synthèse de l'acide N-[cyanoimino(4-cyanophénylamino)méthyl]-L-aspartique N-[(R)-α-méthylbenzyl] α-monoamide :

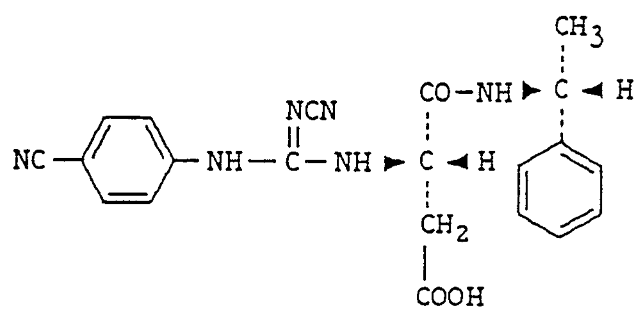

Etape 1 : Préparation de la N-(4-cyanophényl)-N'-cyano-S-méthylisothiourée :

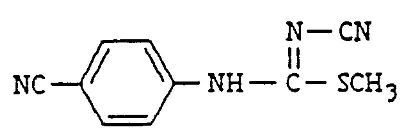

Un mélange de 32 g (0,2 mol) de 4-cyanophényl isothiocyanate et de 12,8 g (0,2 mol) de monosodium cyanamide dans 100 $cm^3$ d'éthanol absolu est maintenu durant 2 heures à l'ébullition. Après refroidissement, le précipité obtenu est filtré et lavé par 200 $cm^3$ d'éthanol absolu. Le solide est alors mis en suspension dans une solution de diméthyl sulfate (25 g, soit 0,2 mol) dans 500 $cm^3$ d'éthanol ; le mélange est chauffé durant 2 heures à ébullition. Le précipité final est filtré, lavé par 2 × 100 $cm^3$ d'eau et par 2 × 100 $cm^3$ d'éthanol, puis est séché sous vide. Il est obtenu 32,8 g (rendement 70 %) d'un solide blanc dont le point de fusion est 220-225 °C.

Etape 2 : un mélange de 2,3 g (0,01 mol) d'acide L-aspartique N-[-(R)-α-méthylbenzyl] α-monoamide, de 3,2 g (0,013 mol) du composé précédemment obtenu et de 0,4 g d'hydroxyde de sodium dans 20 $cm^3$ d'éthanol est chauffé au reflux durant 4 h. Après refroidissement, le solide restant en suspension est éliminé par filtration et le filtrat obtenu est concentré à sec sous vide. Le résidu ainsi obtenu est dissous dans 120 $cm^3$ d'une solution aqueuse de carbonate de sodium à 2 %, puis est lavé par le dichlorométhane (3 × 100 $cm^3$). La phase aqueuse est ensuite acidifiée par une solution de HCl 3N jusqu'à l'obtention d'un pH voisin de 2. Le solide blanc obtenu est filtré, lavé par 2 × 5 $cm^3$ d'eau, puis

séché sous vide. Après recristallisation dans le dichlorométhane, il est obtenu 3,1 g (rendement 70 %) d'un solide blanc dont le point de fusion est de 162 °C.

Le pouvoir sucrant de ce composé est environ 3 000 (trois mille) fois celui du saccharose (sucrose) sur une base molaire par rapport à une solution à 2 % de saccharose, 2 000 (deux mille) fois par rapport à une solution à 5 % et 800 (huit cents) fois par rapport à une solution à 10 % ; l'évaluation a été effectuée dans les mêmes conditions que celles décrites en détail dans la demande de brevet européen EP-A-0 107 597 citée dans le préambule.

Exemple 2

Synthèse du N-[cyanoimino(4-cyanophénylamino) méthyl]-L-aspartyl-L-phénylalanine méthyl ester :

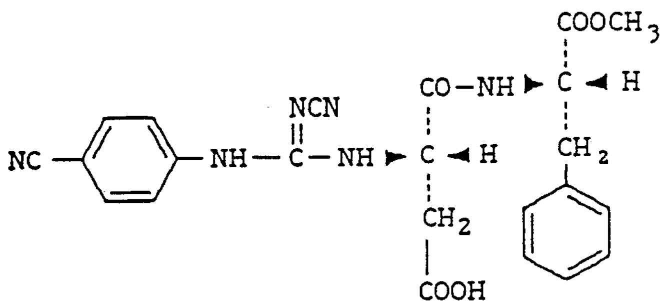

Ce composé, obtenu suivant un protocole identique à celui de l'exemple 1 (rendement 20 % ; point de fusion 173 °C, éthanol) présente une très intense saveur sucrée. Son pouvoir sucrant est environ 40 000 (quarante mille) fois celui du saccharose sur une base molaire par rapport à une solution à 2 % de saccharose, 30 000 (trente mille) fois par rapport à une solution à 5 %, 10 000 (dix mille) fois par rapport à une solution à 10 %.

Exemple 3

Synthèse de l'acide N-[cyanoimino(4-méthoxycarbonylphénylamino)méthyl]-L-aspartique N-[(R)-α-méthylbenzyl] α-monoamide :

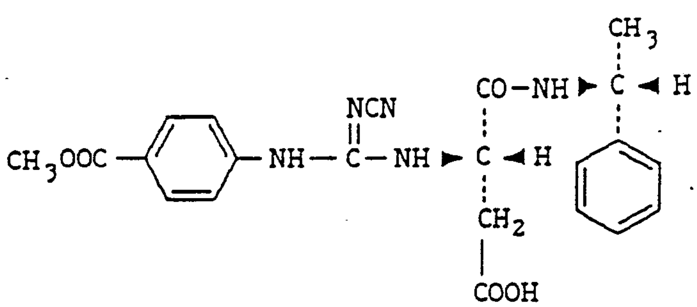

Ce composé, obtenu suivant un protocole identique à celui de l'exemple 1 (rendement 60 % ; point de fusion 96 °C, amorphe) a un pouvoir sucrant qui est environ 1 200 (mille deux cents) fois celui du saccharose sur une base molaire par rapport à une solution à 2 % de saccharose, 800 (huit cents) fois par rapport à une solution à 5 %, 400 (quatre cents) fois par rapport à une solution à 10 %.

Exemple 4

Synthèse de l'acide N-[cyanoimino(4-cyanophénylamino)méthyl]-L-aspartique N-(L-2-isoheptyl)α-monoamide :

(Voir Formule page 6)

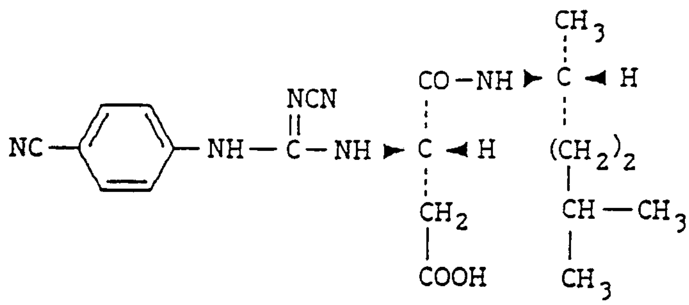

Ce composé, obtenu suivant un protocole identique à celui de l'exemple 1 (rendement 45 % ; point de fusion 170-180 °C, éthanol) a un pouvoir sucrant qui est environ 4 000 (quatre mille) fois celui du saccharose sur une base molaire par rapport à une solution à 2 % de saccharose, 2 500 (deux mille cinq cents) fois par rapport à une solution à 5 %, 1 800 (mille huit cents) fois par rapport à une solution à 10 %.

Exemple 5

Synthèse du N-[cyanoimino(4-cyanophénylamino) méthyl]-L-aspartyl-D-alanine N-(2-butyl)amide :

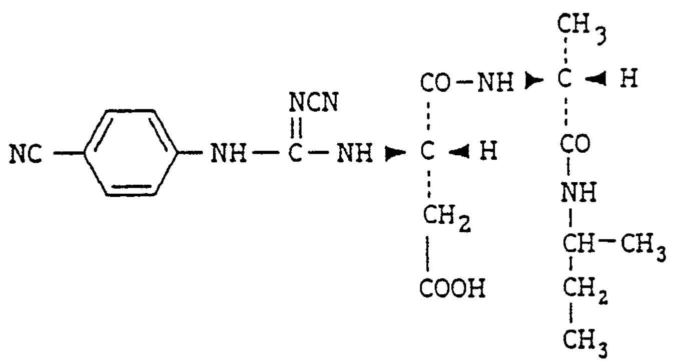

Ce composé, obtenu suivant un protocole identique à celui de l'exemple 1 (rendement 45 % ; point de fusion 187 °C, amorphe), a un pouvoir sucrant qui est environ 800 (huit cents) fois celui du saccharose sur une base molaire par rapport à une solution à 2 % de saccharose.

Les agents édulcorants selon l'invention, présentent de nombreux avantages par rapport aux produits commercialisés à ce jour. On peut citer :

intense pouvoir sucrant ;

faible prix de revient ;

excellente qualité gustative sans arrière-goût amer.

Compte tenu que ces composés sont des édulcorants non nutritifs, non fermentescibles et non cariogènes, ces composés peuvent être utilisés avec succès, seuls ou en association avec d'autres édulcorants, en les ajoutant en quantité efficace, pour édulcorer de nombreux produits, comme par exemple :

comme édulcorants non nutritifs de table (en tablettes, paquets, etc.),

dans les aliments diététiques et basse calorie, comme par exemple dans les boissons, les concentrés de boisson, les boissons instantanées en poudre, dans les cafés, thés, chocolats instantanés préédulcorés, dans les produits laitiers (laits et yogourts, laits en poudre préédulcorés, crèmes fouettées, etc.) ou analogues laitiers, dans les céréales et boissons préédulcorées pour petit déjeuner, dans les desserts (desserts à la gélatine, pudding et autres gâteaux et pâtisseries) et dans les desserts congelés, ice creams et garnitures à la crème fouettée, dans les produits de boulangerie, dans les confitures, marmelades, gelées et miels diététiques, dans les assaisonnements, ketchups, pickles, sauces et autres arômes alimentaires, dans les confiseries (bonbons, bonbons à mâcher, confiseries au chocolat ou au cacao, pâtes de guimauve, etc.),

dans les chewing gums,

dans les dentifrices et les bâtons à lèvres,

dans les bains de bouches et les gargarismes,

dans diverses préparations pharmaceutiques, vétérinaires et cosmétiques (pour améliorer le goût de la préparation ou pour masquer le goût déplaisant de certains produits),

dans divers articles d'hygiène,

dans les tabacs,

dans les aliments pour animaux, etc.

Les agents édulcorants de la présente invention peuvent être avantageusement utilisés sous forme de mélange avec un support compatible, comme par exemple, et ce de manière non limitative, l'amidon et les malto-dextrines, la cellulose, la méthylcellulose, la carboxyméthylcellulose, l'alginate de sodium, les pectines, les gommes, le lactose, le maltose, le glucose, la leucine, le glycérol, le mannitol, le sorbitol, les acides phosphorique, citrique, tartrique, fumarique, benzoïque, sorbique, propionique, leurs sels de sodium, potassium et calcium, le bicarbonate de sodium.

Les agents de la présente invention peuvent aussi être utilisés en association avec d'autres agents édulcorants comme par exemple le saccharose, le sirop de maïs, le fructose, l'aspartame, la glycyrrhizine, le xylitol, l'acésulfame-K, la thaumatine.

**Revendications** (pour les Etats contractants : BE, CH, DE, FR, GB, IT, LI, LU, NL, SE)

1. Agents édulcorants de formule générale :

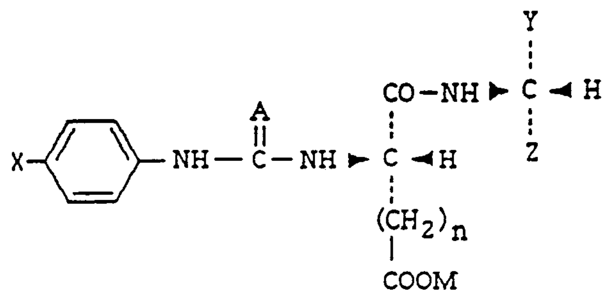

dans laquelle :

X est un substituant CN, $COOC_1$-$C_3$ alkyle, $COC_1$-$C_3$ alkyle, $CONHC_1$-$C_3$ alkyle, $SO_2C_1$-$C_3$ alkyle, $SOC_1$-$C_3$ alkyle, $SO_2NHC_1$-$C_3$ alkyle, $NO_2$, F, Cl ;

n est un nombre égal à 0 ou à 1 ;

M est un atome d'hydrogène, ou un cation organique ou inorganique physiologiquement acceptable ;

Y est un substituant $COOC_1$-$C_3$ alkyle, $C_1$-$C_3$ alkyle, $CH_2OH$, $CHOHCH_3$, $CF_3$ ;

Z est un groupe $C_1$-$C_5$ n-alkyle, isobutyle, isopentyle, phényle, cyclohexyle, benzyle, cyclohexylméthyle, $CH_2C_6H_4OH$(p), $CH_2OC_1$-$C_4$ alkyle, $CH_2COOC_1$-$C_4$ alkyle, $CH_2SC_1$-$C_4$ alkyle, $CH_2CH_2SCH_3$, $CH_2CH_2SO_2CH_3$, $COOC_1$-$C_4$ alkyle, $COOC_3$-$C_7$ cycloalkyle, $CONHC_2$-$C_4$ alkyle, $CONHC_3$-$C_7$ cycloalkyle, $CONHC_3$-$C_7$ thiacycloalkyle, CONH-$CH_2COOCH_3$ ;

caractérisés en ce que A est un groupe N-CN.

2. Agents édulcorants selon la revendication 1, caractérisés en ce que :

X est CN, $COOCH_3$, $COOC_2H_5$, $COCH_3$, $CONHCH_3$, $SO_2CH_3$, $SO_2NHCH_3$ ;

n est égal à 1 ;

M est un atome d'hydrogène ou un cation $Na^+$, $K^+$, $NH_4^+$, 1/2 $Ca^{2+}$, 1/2 $Mg^{2+}$ ;

Y est $COOCH_3$, $CH_3$, $CH_2OH$ ;

Z est un groupe butyle, pentyle, isobutyle, isopentyle, phényle, cyclohexyle, benzyle, cyclohexylméthyle, propylamide, 2-butylamide, dicyclopropylcarbinylamide, 2,2,4,4-tétraméthylthiétan-3-ylamide.

3. Agent édulcorant suivant la revendication 2 caractérisé en ce qu'il consiste en un composé de formule :

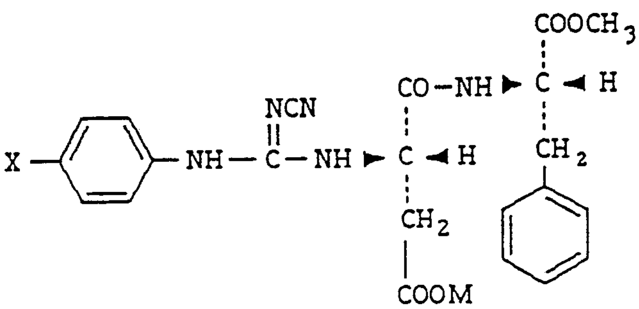

4. Agent édulcorant suivant la revendication 2 caractérisé en ce qu'il consiste en un composé de formule :

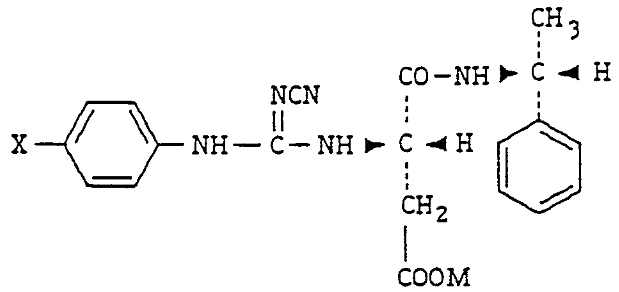

5. Agent édulcorant suivant la revendication 2 caractérisé en ce qu'il consiste en un composé de formule :

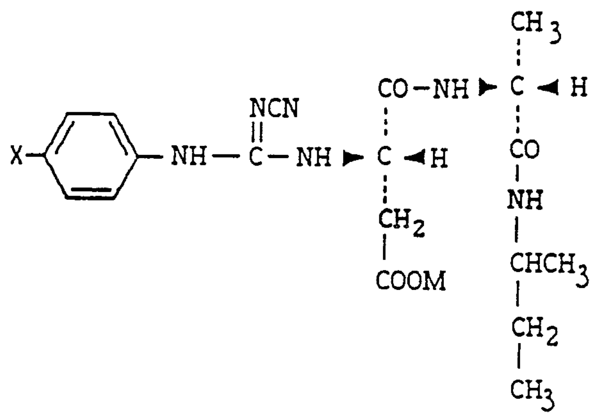

6. Procédé pour édulcorer les aliments, boissons, confiseries, chewing gums, articles de toilette, produits cosmétiques et d'hygiène, préparations pharmaceutiques et vétérinaires, caractérisé en ce qu'il consiste à y ajouter une quantité efficace d'un agent édulcorant selon l'une des revendications 1 à 5.

7. Préparations édulcorées suivant le procédé de la revendication 6.

8. Compositions édulcorantes caractérisées en ce qu'elles comprennent une quantité efficace d'un agent édulcorant selon l'une des revendications 1 à 5 et un support compatible.

9. Compositions édulcorantes selon la revendication 8 caractérisées en ce que le support compatible est choisi dans le groupe comprenant l'amidon et les malto-dextrines, la cellulose, la méthylcellulose et la carboxyméthylcellulose, l'alginate de sodium, les pectines, les gommes, le lactose, le maltose et le glucose, la leucine, le glycérol, le mannitol et le sorbitol, les acides phosphorique, citrique, tartrique, fumarique, benzoïque, sorbique, propionique et leurs sels de sodium, potassium et calcium, le bicarbonate de sodium.

10. Compositions édulcorantes caractérisées en ce qu'elles comprennent un agent édulcorant selon l'une des revendications 1 à 5 et un autre agent édulcorant, l'autre agent édulcorant étant choisi dans le groupe comprenant le saccharose, le sirop de maïs, le fructose, l'aspartame, la glycyrrhizine, le xylitol, l'acésulfame-K, la thaumatine.

**Revendications** (pour l'Etat contractant AT)

1. Procédé pour la préparation d'agents édulcorants de formule générale :

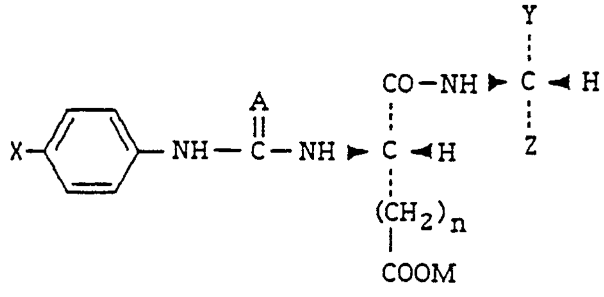

dans laquelle :

X est un substituant CN, $COOC_1$-$C_3$ alkyle, $COC_1$-$C_3$ alkyle, $CONHC_1$-$C_3$ alkyle, $SO_2C_1$-$C_3$ alkyle, $SOC_1$-$C_3$ alkyle, $SO_2NHC_1$-$C_3$ alkyle, $NO_2$, F, Cl ;

n est un nombre égal à 0 ou à 1 ;

M est un atome d'hydrogène, ou un cation organique ou inorganique physiologiquement acceptable ;

Y est un substituant $COOC_1$-$C_3$ alkyle, $C_1$-$C_3$ alkyle, $CH_2OH$, $CHOHCH_3$, $CF_3$ ;

Z est un groupe $C_1$-$C_5$ n-alkyle, isobutyle, isopentyle, phényle, cyclohexyle, benzyle, cyclohexylméthyle, $CH_2C_6H_4OH$(p), $CH_2OC_1$-$C_4$ alkyle, $CH_2COOC_1$-$C_4$ alkyle, $CH_2SC_1$-$C_4$ alkyle, $CH_2CH_2SCH_3$, $CH_2CH_2SO_2CH_3$, $COOC_1$-$C_4$ alkyle, $COOC_3$-$C_7$ cycloalkyle, $CONHC_2$-$C_4$ alkyle, $CONHC_3$-$C_7$ cycloalkyle, $CONHC_3$-$C_7$ thiacycloalkyle, CONH-$CH_2COOCH_3$ ;

A est un groupe N-CN ;

caractérisé en ce qu'il consiste à condenser un premier composé de formule générale :

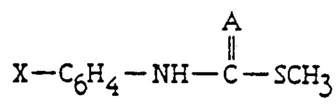

avec un second composé de formule générale :

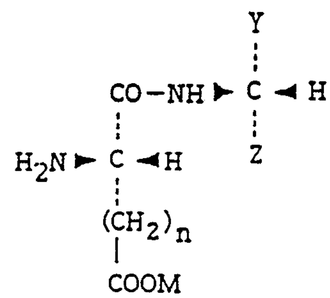

dans lesquels X, n, M, Y, Z et A répondent aux définitions données ci-dessus.

2. Procédé selon la revendication 1, caractérisé en ce que dans les composés utilisés :

X est CN, $COOCH_3$, $COOC_2H_5$, $COCH_3$, $CONHCH_3$, $SO_2CH_3$, $SO_2NHCH_3$ ;

n est égal à 1 ;

M est un atome d'hydrogène ou un cation $Na^+$, $K^+$, $NH_4^+$,1/2 $Ca^{2+}$, 1/2 $Mg^{2+}$ ;

Y est $COOCH_3$, $CH_3$, $CH_2OH$ ;

Z est un groupe butyle, pentyle, isobutyle, isopentyle, phényle, cyclohexyle, benzyle, cyclohexylméthyle, propylamide, 2-butylamide, dicyclopropylcarbinylamide, 2,2,4,4-tétraméthylthiétan-3-ylamide.

3. Procédé selon la revendication 1, caractérisé en ce que la condensation est effectuée en présence d'une base, la base étant choisie dans le groupe comprenant l'hydroxyde de sodium, l'hydroxyde de potassium, la triéthylamine.

4. Procédé selon les revendications 1 et 3 caractérisé en ce que la condensation est effectuée à ébullition dans un mélange éthanol-eau.

5. Procédé de préparation, suivant la revendication 1, d'un agent édulcorant caractérisé en ce qu'il consiste en un composé de formule :

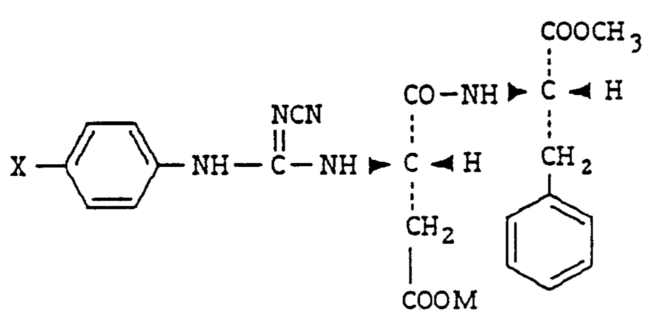

6. Procédé de préparation suivant la revendication 1, d'un agent édulcorant caractérisé en ce qu'il consiste en un composé de formule :

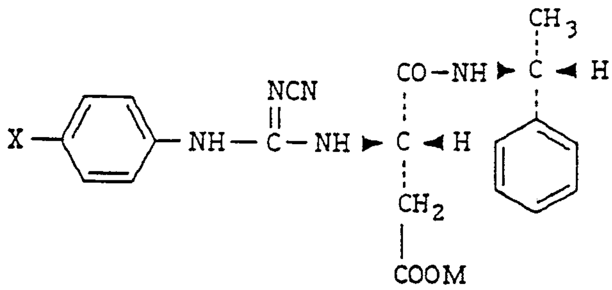

7. Procédé de préparation, suivant la revendication 1, d'un agent édulcorant caractérisé en ce qu'il consiste en un composé de formule :

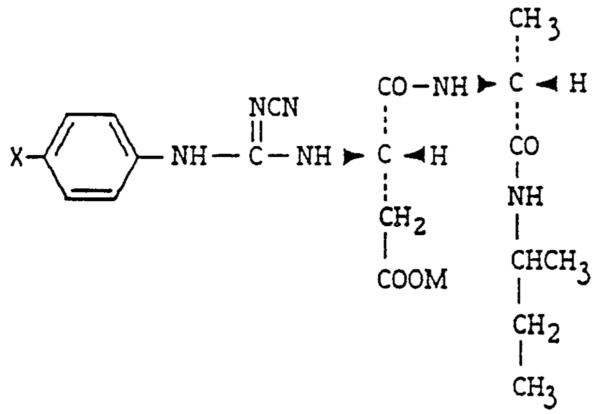

8. Procédé de préparation d'un produit consommable sucré, caractérisé en ce qu'il consiste à ajouter à ce produit une quantité efficace d'un agent édulcorant préparé selon l'une des revendications 1, 2, 5, 6 et 7.

**Claims** (for the Contracting States : BE ; CH ; DE ; FR ; GB ; IT ; LI ; LU ; NL ; SE)

1. Sweetening agents of general formula :

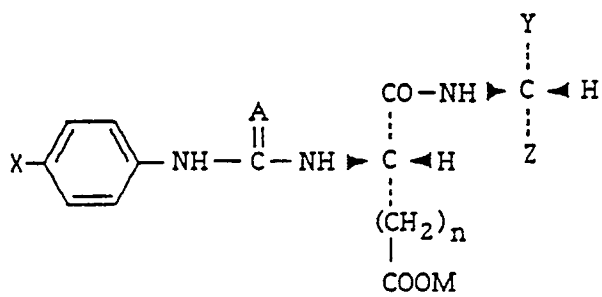

wherein :

X is a CN, $COOC_1$-$C_3$ alkyl, $COC_1$-$C_3$ alkyl, $CONHC_1$-$C_3$ alkyl, $SO_2C_1$-$C_3$ alkyl, $SOC_1$-$C_3$ alkyl, $SO_2NHC_1$-$C_3$ alkyl, $NO_2$, F, Cl substituent ;

n is a number equal to 0 or 1 ;

M is a hydrogen atom, or an organic or inorganic physiologically acceptable cation ;

Y is a $COOC_1$-$C_3$ alkyl, $C_1$-$C_3$ alkyl, $CH_2OH$, $CHOHCH_3$, $CF_3$ substituent ;

Z is a $C_1$-$C_5$-n-alkyl, isobutyl, isopentyl, phenyl, cyclohexyl, benzyl, cyclohexylmethyl, $CH_2C_6H_4OH$(p), $CH_2OC_1$-$C_4$ alkyl, $CH_2COOC_1$-$C_4$ alkyl, $CH_2SC_1$-$C_4$, alkyl, $CH_2CH_2SCH_3$, $CH_2CH_2SO_2CH_3$, $COOC_1$-$C_4$ alkyl, $COOC_3$-$C_7$ cycloalkyl, $CONHC_2$-$C_4$ alkyl, $CONHC_3$-$C_7$ cycloalkyl, $CONHC_3$-$C_7$ thiacycloalkyl, or $CONHCH_2COOCH_3$ group ;

characterized in that A is an N-CN group.

2. Sweetening agents according to claim 1, wherein :
X is CN, $COOCH_3$, $COOC_2H_5$, $COCH_3$, $CONHCH_3$, $SO_2CH_3$, $SO_2NHCH_3$ ;
n is equal to 1 ;
M is a hydrogen atom or a $Na^+$, $K^+$, $NH_4^+$, 1/2 $Ca^{2+}$, 1/2 $Mg^{2+}$ cation ;
Y is $COOCH_3$ ; $CH_3$, $CH_2OH$ ;
Z is a butyl, pentyl, isobutyl, isopentyl, phenyl, cyclohexyl, benzyl, cyclohexylmethyl, propylamide, 2-butylamide, dicyclopropylcarbinylamide, 2,2,4,4-tetramethylthietan-3-ylamide group.

3. Sweetening agent according to claim 2, wherein said agent consists in a compound of formula :

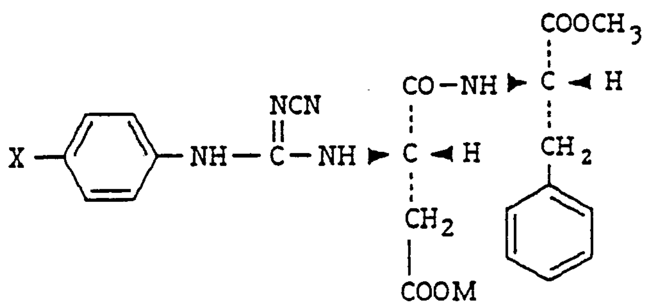

4. Sweetening agent according to claim 2, wherein said agent consists in a compound of formula :

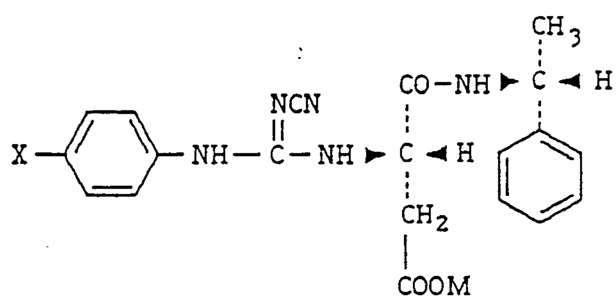

5. Sweetening agent according to claim 2, wherein said agent consists in a compound of formula :

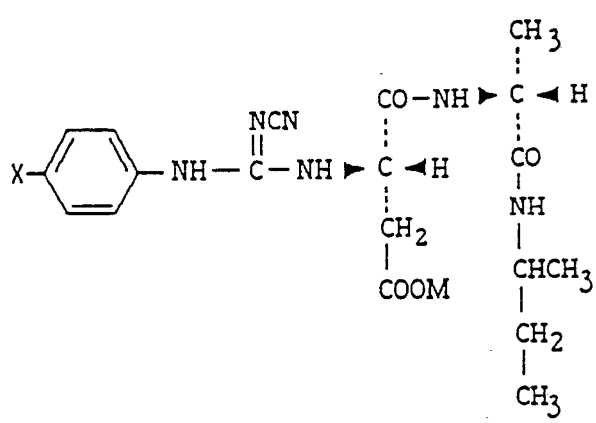

6. Process for sweetening foods, beverages, confectioneries, chewing-gums, toilet articles, cosmetics and hygiene products, pharmaceutical and veterinary preparations, characterized in that said process consists in adding an adequate quantity of a sweetening agent according to any one of claims 1 and 5.

7. Preparations sweetened according to the process of claim 6.

8. Sweetening compositions containing an adequate quantity of a sweetening agent according to any one of claims 1 to 5 with a compatible carrier.

9. Sweetening compositions according to claim 8, wherein the compatible carrier is selected from the group including starch, malto-dextrins, cellulose, methylcellulose, carboxymethylcellulose, sodium alginate, pectins, gums, lactose, maltose, glucose, leucine, glycerol, mannitol, sorbitol, and acids such as phosphoric, citric, tartaric, fumaric, benzoic, sorbic and propionic acids, their sodium, potassium and calcium salts, and sodium bicarbonate.

10. Sweetening compositions wherein said compositions contain a sweetening agent according to any one of claims 1 to 5, with another sweetening agent, which latter is selected from the group including sucrose, corn syrups, fructose, aspartame, glycyrrhizin, xylitol, acesulfam-K, thaumatin.

**Claims** (for the Contracting State AT)

1. A process to prepare sweetening agents of general formula :

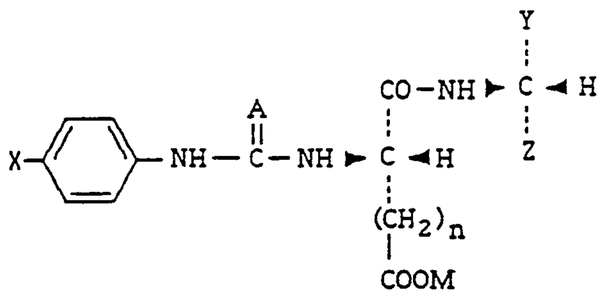

wherein :

X is a CN, $COOC_1$-$C_3$ alkyl, $COC_1$-$C_3$ alkyl, $CONHC_1$-$C_3$ alkyl, $SO_2C_1$-$C_3$ alkyl, $SOC_1$-$C_3$ alkyl, $SO_2NHC_1$-$C_3$ alkyl, $NO_2$, F, Cl substituent ;

n is a number equal to 0 or 1 ;

M is a hydrogen atom, or an organic or inorganic physiologically acceptable cation ;

Y is a $COOC_1$-$C_3$ alkyl, $C_1$-$C_3$ alkyl, $CH_2OH$, $CHOHCH_3$ substituent ;

Z is a $C_1$-$C_5$-n-alkyl, isobutyl, isopentyl, phenyl, cyclohexyl, benzyl, cyclohexylmethyl, $CH_2C_6H_4OH$(p), $CH_2OC_1$-$C_4$ alkyl, $CH_2COOC_1$-$C_4$ alkyl, $CH_2SC_1$-$C_4$ alkyl, $CH_2CH_2SCH_3$ $CH_2CH_2SO_2CH_3$ $COOC_1$-$C_4$ alkyl, $COOC_3$-$C_7$ cycloalkyl, $CONHC_2$-$C_4$ alkyl, $CONHC_3$-$C_7$ cycloalkyl, $CONHC_3$-$C_7$ thiacycloalkyl, or $CONHCH_2COOCH_3$ group ;

A is a N-CN group ;

characterized in that it consists to condensate a first compound of general formula :

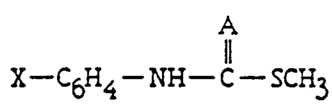

with a second compound of general formula :

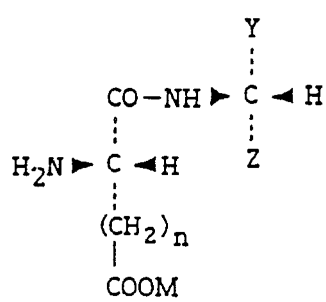

wherein X, n, M, Y, Z and A have the definitions given herein above.

2. A process according to claim 1, characterized in that in the compounds used :

X is CN, $COOCH_3$, $COOC_2H_5$, $COCH_3$, $CONHCH_3$, $SO_2CH_3$, $SO_2NHCH_3$ ;

n is equal to 1 ;
M is a hydrogen atom or a $Na^+$, $K^+$, $NH_4^+$, 1/2 $Ca^{2+}$, 1/2 $Mg^{2+}$ cation ;
Y is $COOCH_3$, $CH_3$, $CH_2OH$ ;
Z is a butyl, pentyl, isobutyl, isopentyl, phenyl, cyclohexyl, benzyl, cyclohexylmethyl, propylamide, 2-butylamide, dicyclopropylcarbinylamide, 2,2,4,4-tetramethylthietan-3-ylamide group.

3. A process according to claim 1, characterized in that the condensation is carried out in the presence of a base, the base being chosen in the group comprising sodium hydroxide, potassium hydroxide, triethylamine.

4. A process according to claims 1 and 3, characterized in that the condensation is carried out in an ethanol-water mixture at boiling point.

5. A process of preparation, according to claim 1, of a sweetening agent characterized in that it consits of a compound of formula :

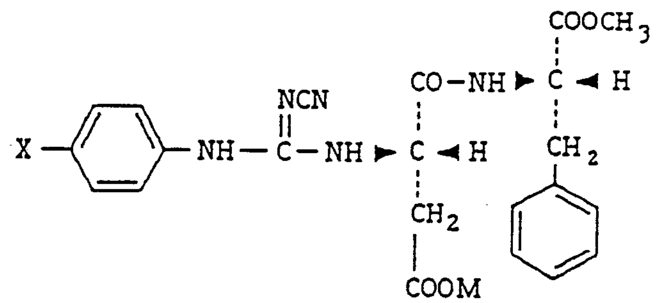

6. A process of preparation, according to claim 1, of a sweetening agent characterized in that it consits of a compound of formula :

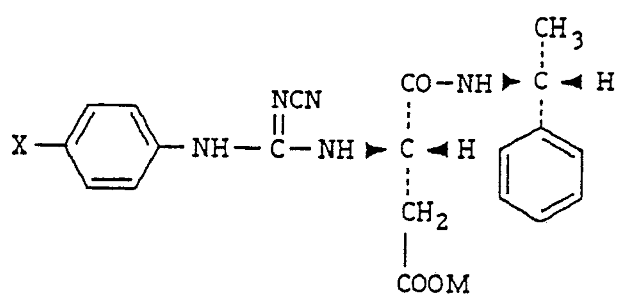

7. A process of preparation, according to claim 1, of a sweetening agent characterized in that it consits of a compound of formula :

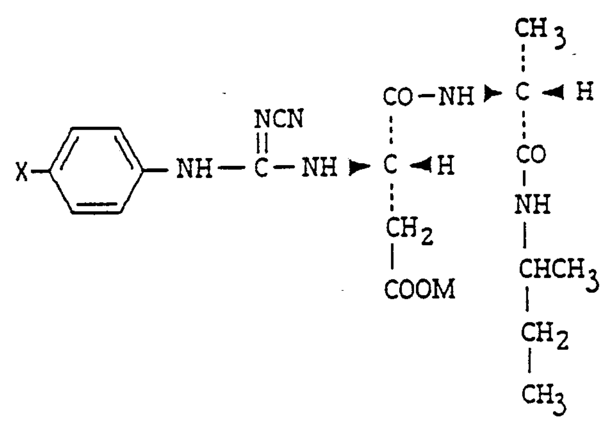

8. A process of preparation of a sweet edible product characterized in that it consists to add to that product an adequate quantity of a sweetening agent prepared according to one of the claims 1, 2, 5, 6 and 7.

**Patentansprüche** (für die Vertragsstaaten : BE ; CH ; DE ; FR ; GB ; IT ; LI ; LU ; NL ; SE)

1. Süßstoffe mit der allgemeinen Formel :

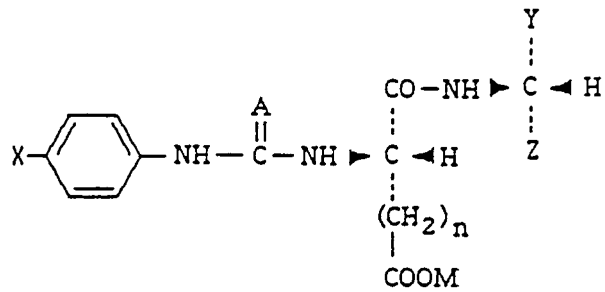

Dabei ist :

X ein Substituent von CN, $COOC_1$-$C_3$ alkyl, $COC_1$-$C_3$ alkyl, $CONHC_1$-$C_3$ alkyl, $SO_2C_1$-$C_3$ alkyl, $SOC_1$-$C_3$ alkyl, $SO_2NHC_1$-$C_3$ alkyl, $NO_2$, F, Cl ;

n eine Zahl mit dem Wert 0 oder 1 ;

M ein Sauerstoffatom oder ein physiologisch verträgliches organisches bzw. anorganisches Kation ;

Y ein Substituent von $COOC_1$-$C_3$ alkyl, $C_1$-$C_3$ alkyl, $CH_2OH$, $CHOHCH_3$, $CF_3$ ;

Z eine der folgenden Gruppen : $C_1$-$C_5$ n-alkyl, isobutyl, isopentyl, phenyl, cyclohexyl, benzyl, cyclohexylmethyl, $CH_2C_6H_4OH$(p), $CH_2OC_1$-$C_4$ alkyl, $CH_2COOC_1$-$C_4$ alkyl, $CH_2SC_1C_4$ alkyl, $CH_2CH_2SCH_3$, $CH_2CH_2SO_2CH_3$, $COOC_1$-$C_4$ alkyl, $COOC_3$-$C_7$ cycloalkyl, $CONHC_2$-$C_4$ alkyl, $CONHC_3$-$C_7$ cycloalkyl, $CONHC_3$-$C_7$ thiacycloalkyl, CONH-$CH_2COOCH_3$ ;

dadurch gekennzeichnet, daß A eine N-CN-Gruppe ist.

2. Süßstoffe nach dem Anspruch 1, dadurch gekennzeichnet, daß :

X für CN, $COOCH_3$, $COOC_2H_5$, $COCH_3$, $CONHCH_3$, $SO_2CH_3$, $SO_2NHCH_3$ steht ;

n dem Wert 1 entspricht ;

M ein Wasserstoffatom oder eines der folgenden Kationen ist : $Na^+$, $K^+$, $NH_4^+$, 1/2 $Ca^{2+}$, 1/2 $Mg^{2+}$ ;

Y für $COOCH_3$, $CH_3$, $CH_2OH$ steht ;

Z eine der folgenden Gruppen ist : butyl, pentyl, isobutyl, isopentyl, phenyl, cyclohexyl, benzyl, cyclohexylmethyl, propylamid, 2-butylamid, dicyclopropylcarbinylamid, 2,2,4,4-tetramethylthietan-3-ylamid.

3. Süßstoffe nach dem Anspruch 2, dadurch gekennzeichnet, daß er aus einer Verbindung mit foldenger Formel besteht :

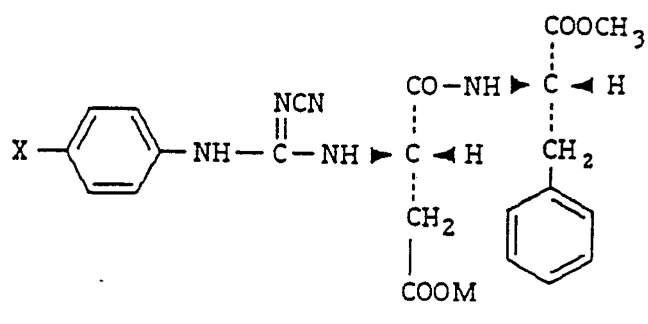

4. Süßstoffe nach dem Anspruch 2, dadurch gekennzeichnet, daß er aus einer Verbindung mit foldenger Formel besteht :

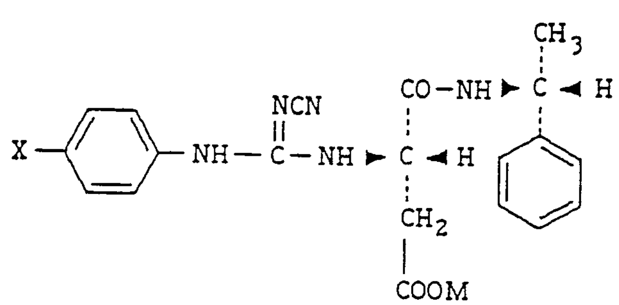

5. Süßstoffe nach dem Anspruch 2, dadurch gekennzeichnet, daß er aus einer Verbindung mit foldenger Formel besteht :

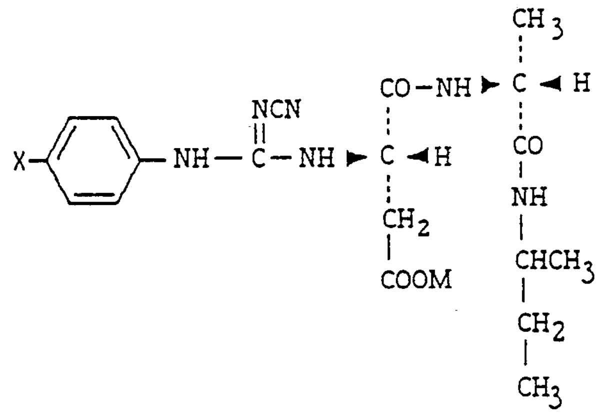

6. Verfahren zum Süßen von Nahrungsmitteln, Getränken, Süßwaren, Kaugummis, Toilettenartikeln, Kosmetik- und Hygieneprodukten, pharmazeutischen und veterinärmedizinischen Präparaten, dadurch gekennzeichnet, daß dabei eine wirksame Menge eines Süßstoffs nach einem der Ansprüche 1-5 hinzugegeben wird.

7. Süßpräparate nach dem Verfahren des Ansprüche 6.

8. Süßstoffzusammensetzungen, dadurch gekennzeichnet, dab sie eine wirksame Menge eines Süßstoffes nach einem der Ansprüche 1-5 und einen kompatiblen Träger enthalten.

9. Süßstoffzusammensetzungen nach dem Ansprüch 8, dadurch gekennzeichnet, daß kompatible Träger aus der folgenden Gruppe stammt : Stärke und Malzdextrine, Zellulose, Methylzellulose und carboximethylzellulose, Natriumalginat, Pektine, Gummis, Lactose, Maltose und Glucose, Leucin, Glycerol, Mannit und Sorbit, Phosphor-, Zitronen-, Wein-, Fumar-, Benzoe-, Sorbin- und Propionsäure und deren Natrium-, Kalium- und Kalziumsalze, Natriumbikarbonat.

10. Süßstoffzusammensetzungen, dadurch gekennzeichnet, daß sie einen Süßstoff nach einem der Ansprüche 1-5 und einen anderen Süßstoff enthalten, wobei der andere Süßstoff aus folgender Gruppe stammt : Saccharose, Maissirup, Fructose, Aspartam, Glycyrrhizin, Pentol, Acesufalm-K, Thaumatin.

**Patentansprüche** (für den Vertragsstaat AT)

1. Verfahren für die Zubereitung von Süßstoffen mit der allgemeinen Formel :

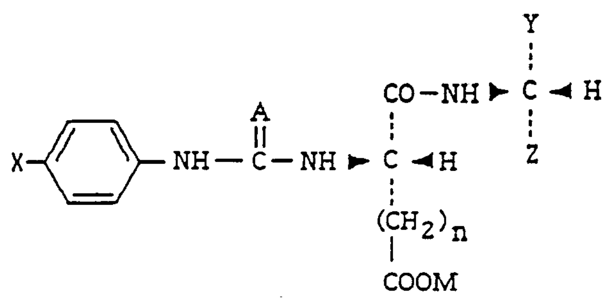

Dabei ist :

X ein Substituent von CN, $COOC_1$-$C_3$ alkyl, $COC_1$-$C_3$ alkyl, $CONHC_1$-$C_3$ alkyl, $SO_2C_1$-$C_3$ alkyl, $SOC_1$-$C_3$ alkyl, $SO_2NHC_1$-$C_3$ alkyl, $NO_2$, F, Cl ;

n eine Zahl mit dem Wert 0 oder 1 ;

M ein Sauerstoffatom oder ein physiologisch verträgliches organisches bzw. anorganisches Kation ;

Y ein Substituent von $COOC_1$-$C_3$ alkyl, $C_1$-$C_3$ alkyl, $CH_2OH$, $CHOHCH_3$, $CF_3$ ;

Z eine der folgenden Gruppen : $C_1$-$C_5$ n-alkyl, isobutyl, isopentyl, phenyl, cyclohexyl, benzyl, cyclohexylmethyl, $CH_2C_6H_4OH$(p), $CH_2OC_1$-$C_4$ alkyl, $CH_2COOC_1$-$C_4$ alkyl, $CH_2SC_1$-$C_4$ alkyl, $CH_2CH_2SCH_3$, $CH_2CH_2SO_2CH_3$, $COOC_1$-$C_4$ alkyl, $COOC_3$-$C_7$ cycloalkyl, $CONHC_2$-$C_4$ alkyl, $CONHC_3$-$C_7$ cycloalkyl, $CONHC_3$-$C_7$ thiacycloalkyl, CONH-$CH_2COOCH_3$ ;

A eine N-CN-Gruppe ;
dadurch gekennzeichnet, daß eine erste Verbindung mit der allgemenein Formel :

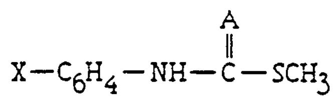

mit einer zweiten Verbindung mit der allgemeinen Formel :

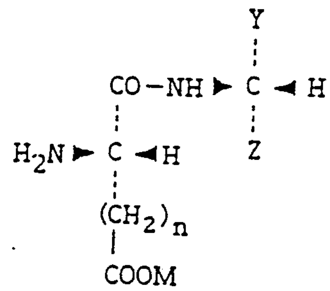

kondensiert wird, wobei X, n, M, Y, Z und A den oben genannten Definitionen entsprechen.

2. Verfahren nach dem Anspruch 1, dadurch gekennzeichnet, daß bei den verwendeten Verbindungen :
X für CN, $COOCH_3$, $COOC_2H_5$, $COCH_3$, $CONHCH_3$, $SO_2CH_3$, $SO_2NHCH_3$ steht ;
n dem Wert 1 entspricht ;
M ein Wasserstoffatom oder eines der folgenden Kationen ist : $Na^+$, $K^+$, $NH_4^+$, 1/2 $Ca^{2+}$, 1/2 $Mg^{2+}$ ;
Y für $COOCH_3$, $CH_3$, $CH_2OH$ steht ;
Z eine der folgenden Gruppen ist : butyl, pentyl, isobutyl, isopentyl, phenyl, cyclohexyl, benzyl, cyclohexylmethyl, propylamid, 2-butylamid, dicyclopropylcarbinylamid, 2,2,4,4-tetramethylthietan-3-ylamid.

3. Verfahren nach dem Anspruch 1, dadurch gekennzeichnet, daß die Kondensation in Anwesenheit einer Base erfolgt, wobei die Kaliumhydroxid, Triäthylamin.

4. Verfahren nach den Ansprüchen 1 und 3, dadurch gekennzeichnet, daß die Kondensation in einem kochenden Äthanol-Wasser-Gemisch erfolgt.

5. Zubereitungsverfahren eines Süßstoffes nach dem Anspruch 1, dadurch gekennzeichnet, daß er aus einer Verbindung mit der folgenden Formel besteht :

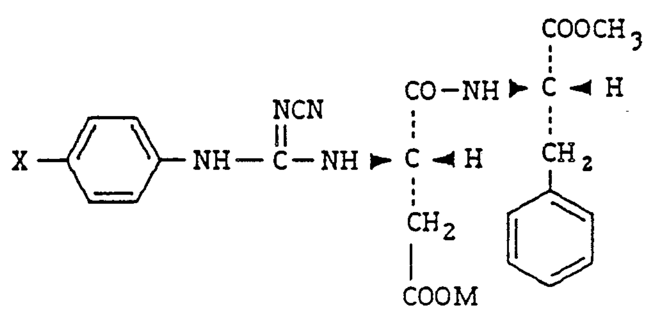

6. Zubereitungsverfahren eines Süßstoffes nach dem Anspruch 1, dadurch gekennzeichnet, daß er aus einer Verbindung mit der folgenden Formel besteht :

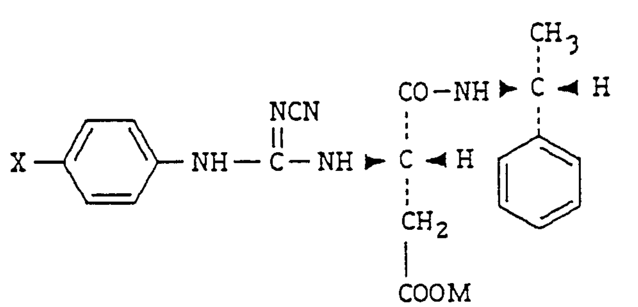

7. Zubereitungsverfahren eines Süßstoffes nach dem Anspruch 1, dadurch gekennzeichnet, daß er aus einer Verbindung mit der folgenden Formel besteht :

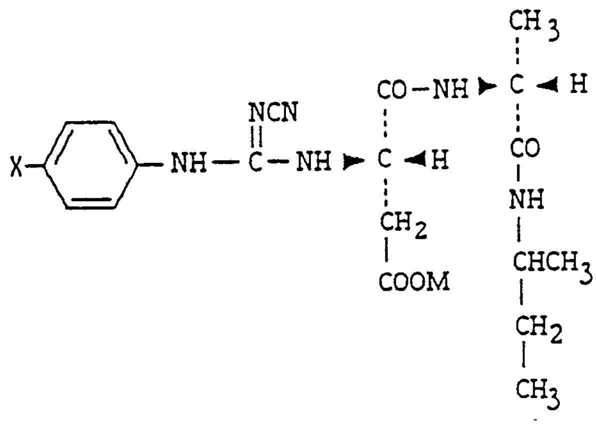

8. Zubereitungsverfahren eines zuckerhaltigen verzehrbaren Produktes, dadurch gekennzeichnet, daß dabei diesem Produkt eine wirksame Menge eine Süßstoffes nach einem der Ansprüche 1, 2, 5, 6, und 7 zugegeben wird.